# EUROPÄISCHE PATENTANMELDUNG

(11) **EP 4 717 298 A1**
(43) Veröffentlichungstag der Anmeldung: **01.04.2026**
(21) Anmeldenummer: 25200530.1
(22) Anmeldetag: 05.09.2025
(51) Int. Cl.: A61M 25/00, A61M 25/01, A61M 25/06

(54) **EINFÜHRINSTRUMENT FÜR KATHETER**

(30) Priorität: 30.09.2024 DE 202024105631 U
(71) Anmelder: Spiggle & Theis Medizintechnik GmbH, 51491 Overath (DE)
(72) Erfinder: ARSLAN-STEUTERMANN, Murat, 51491 Overath (DE)
(74) Vertreter: Seyer & Nobbe Patentanwälte PartmbB

(57) **Zusammenfassung**

Einführinstrument für Katheter umfassend einen ersten länglichen Hohlprofilkörper mit einem distalen und einem proximalen Ende, einen zweiten länglichen Hohlprofilkörper, der teilweise in den ersten länglichen Hohlprofilkörper eingeführt ist, der verschiebbar in dem ersten länglichen Hohlprofilkörper gelagert ist und der sich über das proximale Ende des ersten länglichen Hohlprofilkörpers hinaus erstreckt, und ein sich an das distale Ende des ersten länglichen Hohlprofilkörpers anschließendes Rohr, wobei der zweite längliche Hohlprofilkörper von einer ersten Stellung in eine zweite Stellung verschiebbar ist, in welcher der zweite längliche Hohlprofilkörper weiter in den ersten länglichen Hohlprofilkörper eingeführt ist als in der ersten Stellung, wobei in der ersten Stellung ein weiteres Einführen des zweiten länglichen Hohlprofilkörpers in den ersten länglichen Hohlprofilkörpers gehemmt ist.

## Beschreibung

Die vorliegende Erfindung betrifft ein Einführinstrument für Katheter, insbesondere Ballonkatheter. Derartige Katheter werden üblicherweise verwendet, um Gefäße oder Röhren, wie beispielsweise die Eustachi-Röhre, zu weiten. Ballonkatheter weisen hierzu üblicherweise am distalen Ende ein Ballon auf, welcher mittels einer vom proximalen Ende einfüllbaren Lösung expandiert werden kann, sobald er die Engstelle erreicht hat.

Bekannt sind Einführvorrichtungen für Katheter, die aus einem Griff und einem daran anschließenden Rohr bestehen. Diese Vorrichtungen können durch eine Körperöffnung, wie die Nase oder den Mund, in den Körper eingeführt werden, bis das distale Rohrende unmittelbar vor der Engstelle positioniert ist. Dieser Vorgang kann mithilfe eines Endoskops überwacht werden. Danach wird ein Dilatationskatheter durch den Griff und das Rohr bis zur Engstelle in dem Körper geschoben. Es gibt auch Einführvorrichtungen, die bereits einen Katheter enthalten, sodass die Vorrichtung zusammen mit dem Katheter durch die Körperöffnung eingeführt wird.

Ein Nachteil der bekannten Einführvorrichtungen ist, dass sie die gleichzeitige Verwendung beider Hände des Anwenders erfordern: Eine Hand muss die Vorrichtung positionieren, während die andere den Katheter vorschiebt. Zudem ist es schwierig, den Katheter präzise in der gewünschten Länge in die Engstelle zu schieben. Bei Einführvorrichtungen, die bereits einen Katheter enthalten, besteht die Gefahr, dass der Katheter vor Erreichen der Engstelle distal oder proximal aus der Einführvorrichtung herausgleitet.

Die Aufgabe der Erfindung ist, die aus dem Stand der Technik bekannten Nachteile zu überwinden. Als Aufgabe der Erfindung kann somit unter anderem angesehen werden, ein Einführinstrument bereitzustellen, das einhändig zu bedienen ist.

Zudem soll ein unbeabsichtigtes Herausrutschen des Katheters, insbesondere aus dem distalen Ende des Einführinstrumentes, verhindert werden.

Zur Lösung der Aufgabe ist ein Einführinstrument für Katheter vorgesehen, das einen ersten länglichen Hohlprofilkörper mit einem distalen und einem proximalen Ende, einen zweiten länglichen Hohlprofilkörper, der teilweise in den ersten länglichen Hohlprofilkörper eingeführt ist, der verschiebbar in dem ersten länglichen Hohlprofilkörper gelagert ist und der sich über das proximale Ende des ersten länglichen Hohlprofilkörpers hinaus erstreckt, und ein sich an das distale Ende des ersten länglichen Hohlprofilkörpers anschließendes Rohr umfasst, wobei der zweite längliche Hohlprofilkörper von einer ersten Stellung in eine zweite Stellung verschiebbar ist, in welcher der zweite längliche Hohlprofilkörper weiter in den ersten länglichen Hohlprofilkörper eingeführt ist als in der ersten Stellung, wobei in der ersten Stellung ein weiteres Einführen des zweiten länglichen Hohlprofilkörpers in den ersten länglichen Hohlprofilkörpers gehemmt ist.

Das Einführinstrument weist somit zwei zumindest teilweise ineinandergesteckte Hohlprofilkörper auf. Am distalen Ende des einen Hohlprofilkörpers ist ein Rohr angeschlossen. Im Inneren des Einführinstruments wird somit Kanal ausgebildet, der vom proximalen Ende des Einführinstruments bis zum distalen Ende verläuft. Durch diesen Kanal kann der Katheter geführt werden. Die beiden Hohlprofilkörper sind so ausgestaltet, dass sie relativ zueinander beweglich sind. Die Hohlprofilkörper können rohrförmig ausgebildet sein.

Der zweite Hohlprofilkörper ist in einer ersten Stellung nicht vollständig in den ersten Hohlprofilkörper eingeschoben. Das proximale Ende des zweiten Hohlprofilkörpers erstreckt sich somit über das proximale Ende des ersten Hohlprofilkörpers hinaus. Von dieser ersten Stellung kann der zweite Hohlprofilkörper in eine zweite Stellung geschoben werden, in welcher der zweite Hohlprofilkörper weiter in den ersten Hohlprofilkörper eingeführt ist. Das heißt, dass in der zweiten Stellung des zweiten Hohlprofilkörpers nur noch ein geringerer Teil des proximalen Endes des zweiten Hohlprofilkörpers sich über das proximale Ende des ersten Hohlprofilkörpers erstreckt.

Durch das Einschieben des zweiten Hohlprofilkörpers in den ersten kann ein in dem Einführinstrument befindlicher Katheter ebenfalls verschoben werden. In der ersten Stellung ragt das distale Katheterende nicht über das distale Ende des Einführinstruments hinaus, während es in der zweiten Stellung über das distale Ende hinausragt.

In der ersten Stellung ist ein weiteres Einführen des zweiten länglichen Hohlprofilkörpers in den ersten länglichen Hohlprofilkörpers gehemmt. Von der ersten Stellung aus kann der zweite Hohlprofilkörper somit nicht ohne Kraftaufwand weiter den ersten Hohlprofilkörper verschoben werden. Die Hemmung verhindert also eine unbeabsichtigte Bewegung des zweiten Hohlprofilkörpers und dient als Positionssicherung. Auf diese Weise kann dafür gesorgt werden, dass der zweite Hohlprofilkörper und damit auch ein innerhalb des Einführinstruments angeordneter Katheter in einer festen Position bleibt, wodurch ein unbeabsichtigtes Herausrutschen des Katheters verhindert wird. Erst nach dem gezielten überwinden des Widerstandes, also der Hemmung, kann der zweite Hohlprofilkörper weiter in den ersten Hohlprofilkörper eingeführt werden, um den Katheter aus dem distalen Ende hinaus in die Körperengstelle zu schieben.

Das Verschieben der beiden Hohlprofilkörper ist somit in der ersten Stellung lediglich gehemmt, also erschwert, aber nicht vollständig blockiert. Diese Hemmung kann durch Kraftaufwand überwunden werden. Die Hemmung sorgt somit dafür, dass die Bewegung bewusst und präzise ausgeführt wird, sodass unbeabsichtigte Bewegungen verhindert werden. Dahingegen würde eine Blockierung eine Bewegung vollständig verhindern.

In einer vorteilhaften Ausgestaltung der Erfindung ist in der ersten Stellung ein weiteres Herausziehen des zweiten länglichen Hohlprofilkörpers aus dem ersten länglichen Hohlprofilkörper gehemmt. Somit ist vorzugsweise eine Bewegung des zweiten Hohlprofilkörpers in beide Richtungen, also weiter in den ersten Hohlprofilkörper hinein sowie weiter aus dem ersten Hohlprofilkörper hinaus, gehemmt. Auf diese Weise kann sichergestellt werden, dass ein in dem Einführinstruments befindlichen Katheter in der vorgesehenen Position verbleibt.

Vorzugsweise ist in der zweiten Stellung ein weiteres Einführen des zweiten länglichen Hohlprofilkörpers in den ersten länglichen Hohlprofilkörpers blockiert. Auf diese Weise kann sichergestellt werden, dass der zweite Hohlprofilkörper nur eine vorgegebene Distanz, also die Länge zwischen der ersten Stellung und der zweiten Stellung, in den ersten Hohlprofilkörper eingeschoben werden kann. So kann erreicht werden, dass ein Katheter in dem Einführinstrument nur um diese vorgesehene Länge vorgeschoben werden kann. Der Katheter kann somit nur in der gewünschten Länge in die Engstelle geschoben werden, wobei der Anwender dies nicht genau, beispielsweise mittels eines Endoskops anhand von Farbmarkern auf dem Katheter, überprüfen muss, sondern die Hohlprofilkörper bis zum Anschlag ineinander schieben kann.

Vorzugsweise wird die Hemmung in der ersten Stellung durch eine Hemmvorrichtung erreicht. Die Hemmvorrichtung kann eine Wechselwirkung, bevorzugt eine Reibwirkung, zwischen dem ersten und dem zweiten länglichen Hohlprofilkörper erzeugen. Die Bewegung des zweiten Hohlprofilkörpers im ersten Hohlprofilkörper kann somit durch eine gezielte mechanische Wechselwirkung zwischen den beiden Körpern verlangsamt oder erschwert werden. Diese Wechselwirkung erfolgt vorzugsweise durch Reibung.

Die Hemmvorrichtung kann innerhalb des ersten länglichen Hohlprofilkörpers angeordnet sein. Vorzugsweise umfasst die Hemmvorrichtung eine erste Erhebung auf einem der Hohlprofilkörper, die in der ersten Stellung gegen den anderen Hohlprofilkörper drückt, wodurch der gewünschte Widerstand erreicht wird. Durch erhöhten Kraftaufwand kann der Hohlprofilkörper mit der Erhebung gegen den Widerstand relativ zum anderen Hohlprofilkörper bewegt werden.

Der Hohlprofilkörper, gegen den die erste Erhebung des anderen länglichen Hohlprofilkörpers drückt, kann eine Nut oder einen Schlitz zur Aufnahme der ersten Erhebung nach dem Überwinden des Widerstands aufweisen. Nachdem die Hemmung überwunden wurde, also der Hohlprofilkörper mit der Erhebung gegen den Widerstand verschoben wurde, kann die Erhebung in eine Nut, einen Schlitz oder eine sonstige Aussparung eingeführt werden, sodass die Hemmwirkung aufgehoben ist und die beiden Hohlprofilkörper leichtgängig weiter ineinander geschoben werden können. Wie zuvor ausgeführt, kann bei Erreichen einer zweiten Stellung das weitere Ineinanderschieben blockiert sein. Vorzugsweise bildet die erste Erhebung in Richtung des proximalen Endes des Hohlprofilkörpers einen Keil aus, dessen Spitze in Richtung des proximalen Endes zeigt. Auf diese Weise können die Hohlprofilkörper leicht in die erste Stellung gebracht werden.

Vorzugsweise ist die erste Erhebung an dem zweiten länglichen Hohlprofilkörper angeordnet ist. Vorzugsweise ist die Nut, der Schlitz oder die sonstige Aussparung an dem ersten länglichen Hohlprofilkörper angeordnet. Die erste Erhebung ist folglich vorzugsweise an der Außenseite des zweiten Hohlprofilkörpers angeordnet.

In einer vorteilhaften Ausgestaltung umfassend das Einführinstrument eine zweite Erhebung auf einem der Hohlprofilkörper. Diese zweite Erhebung kann in der ersten Stellung der Hohlprofilkörper ein weiteres Herausziehen des zweiten länglichen Hohlprofilkörpers aus dem ersten länglichen Hohlprofilkörper hemmen. So kann sichergestellt werden, dass der zweite Hohlprofilkörper nicht aus dem ersten Hohlprofilkörper rutschen kann, sondern nur eine vorgegebene Länge herausgezogen werden kann. In der ersten Stellung ist die zweite Erhebung vorzugsweise am proximalen Ende des Schlitzes, der Nut oder der sonstigen Aussparung angeordnet.

Die zweite Erhebung kann distal zur ersten Erhebung angeordnet sein. Vorzugsweise ist die zweite Erhebung auf dem länglichen Hohlprofilkörper angeordnet, der ebenfalls die erste Erhebung aufweist. Vorzugsweise ist die zweite Erhebung höher ausgebildet als die erste Erhebung. Auf diese Weise kann sichergestellt werden, dass der zweite Hohlprofilkörper nicht über die erste Stellung hinaus aus dem ersten Hohlprofilkörper gezogen werden kann.

Vorzugsweise ist die erste und/oder zweite Erhebung elastisch gelagert. Bevorzugt weist der längliche Hohlprofilkörper im Bereich einer Erhebung eine oder mehrere Aussparungen auf. Diese Aussparungen machen den Hohlprofilkörper in diesem Bereich flexibler, sodass er leichter verformt werden kann. Dadurch lässt sich die Kraft, die zum Überwinden des Widerstands in der ersten Stellung erforderlich ist, durch Variation der Aussparungen gezielt anpassen.

In einer vorteilhaften Ausgestaltung weist der zweite längliche Hohlprofilkörper ein Blockierelement auf. Das Blockierelement kann in der zweiten Stellung ein weiteres Einführen des zweiten länglichen Hohlprofilkörpers in den ersten länglichen Hohlprofilkörper blockieren. Vorzugsweise ist das Blockierelement außerhalb des ersten länglichen Hohlprofilkörpers angeordnet. Das Blockierelement kann einen anderen, vorzugsweise einen größeren, Querschnitt aufweisen, als der Teil des zweiten länglichen Hohlprofilkörpers, der vorgesehen ist, in den ersten länglichen Hohlprofilkörper eingeführt zu werden. Somit kann der zweite längliche Hohlprofilkörper soweit in den ersten länglichen Hohlprofilkörper eingeführt werden, bis der veränderte Querschnitt ein weiteres Einschieben verhindert.

Der zweite längliche Hohlprofilkörper kann einen Daumenring aufweisen, der das Einführen des zweiten Hohlprofilkörpers in den ersten erleichtert. Der Anwender kann den Daumen durch den Daumenring führen, um den zweiten Hohlprofilkörper weiter in den ersten zu drücken.

Der erste längliche Hohlprofilkörper kann mindestens einen Fingerring aufweisen. Vorzugsweise umfasst er zwei Fingerringe, durch die beispielsweise der Zeige- und Mittelfinger geführt werden können, um während des Einschiebens des zweiten Hohlprofilkörpers Gegendruck auszuüben.

Vorzugsweise ist der zweite längliche Hohlprofilkörper ausgestaltet, einen Katheter in sich aufzunehmen und einen aufgenommenen Katheter derart zu fixieren, dass dieser beim Verschieben des zweiten länglichen Hohlprofilkörpers relativ zum ersten länglichen Hohlprofilkörpers mitgeführt wird. Dadurch wird sichergestellt, dass der Katheter exakt um die Länge bewegt wird, um die sich der zweite Hohlprofilkörper relativ zum ersten bewegt.

Das am distalen Ende des ersten länglichen Hohlprofilkörpers anschließende Rohr kann gekrümmt sein. Gebogene Rohre haben sich als vorteilhaft erwiesen, um verschiedene Gefäße oder Röhren, wie zum Beispiel die Eustachi-Röhre, zu erreichen.

Das am distalen Ende des ersten länglichen Hohlprofilkörpers anschließende Rohr kann eine Verjüngung zum distalen Ende hin aufweisen. Auf diese Weise kann erreicht werden, dass die Positionierung des Einführinstruments mittels eines Endoskops überwacht werden kann, das beispielsweise durch dieselbe Körperöffnung eingeführt wurde.

Das am distalen Ende des ersten länglichen Hohlprofilkörpers anschließende Rohr kann eine distale Olive aufweisen. Diese Olive kann mit einer Markierung versehen sein, vorzugsweise einer Farb- und/oder Laser-Markierung. Dadurch lässt sich die Position des distalen Endes des Einführinstruments im Körper, beispielsweise mittels eines Endoskops, einfach bestimmen. Insbesondere kann so sichergestellt werden, dass das Einführinstrument nicht zu weit in den Körper, insbesondere in die zu weitende Engstelle, vorgeschoben wird.

Die Erfindung ist zudem auf das erfindungsgemäße Einführinstrument gerichtet, das einen Katheter, insbesondere einen Ballonkatheter, umfasst.

Die Erfindung wird im Folgenden anhand der Figuren nochmals beispielhaft erläutert.

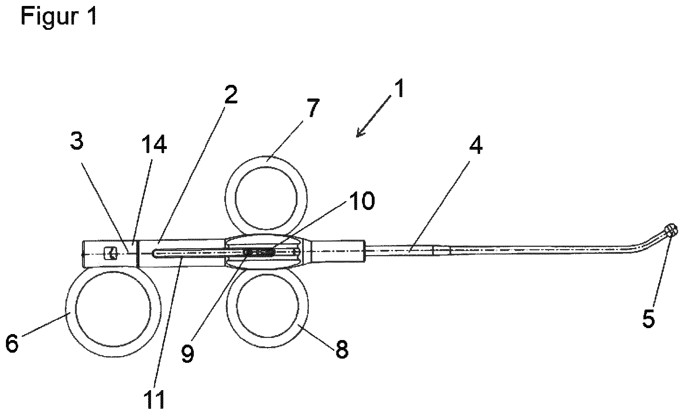

Fig. 1 zeigt eine beispielhafte Ausführungsform eines erfindungsgemäßen Einführinstruments 1 umfassend einen ersten länglichen Hohlprofilkörper 2, in dessen Hohlprofilkörper von der proximalen Seite einen zweiter länglicher Hohlprofilkörper 3 teilweise eingeschoben ist. An der distalen Seite des ersten länglichen Hohlprofilkörpers 2 schließt sich ein Rohr 4 mit einer Olive 5 am distalen Ende an. Der Teil des zweiten Hohlprofilkörpers 3, der sich innerhalb des Hohlprofils des ersten länglichen Hohlprofilkörpers 2 befindet, weist an seiner Außenseite eine erste Erhebung 9 und eine zweite Erhebung 10 auf. Der erste längliche Hohlprofilkörper 2 und der zweite längliche Hohlprofilkörper 3 sind derart zueinander beweglich ausgestaltet, dass der zweite längliche Hohlprofilkörper 3 weiter in den ersten länglichen Hohlprofilkörper 2 eingeschoben werden kann. Beim Einschieben des zweiten länglichen Hohlprofilkörpers 3 in den ersten länglichen Hohlprofilkörper 2 erstrecken sich die Erhebungen 9, 10 durch den Schlitz 11 nach außen, sodass diese nicht gegen die Innenseite des ersten länglichen Hohlprofilkörpers 2 drücken und das Einschieben hemmen. Der zweite längliche Hohlprofilkörper 3 kann so weit aus dem ersten länglichen Hohlprofilkörper 2 hinausgezogen werden, dass die erste Erhebung 9 nicht mehr im Bereich des Schlitzes 11 angeordnet ist, sondern gegen die Innenseite des ersten länglichen Hohlprofilkörpers 2 drückt Hierdurch ist in dieser Position, der ersten Stellung, das Einschieben des zweiten länglichen Hohlprofilkörpers 3 in den ersten länglichen Hohlprofilkörper 2 gehemmt. In dieser ersten Stellung liegt die zweite Erhebung 10 am Ende des Schlitzes 11 an. In der Fig. 1 ist der zweite längliche Hohlprofilkörper 3 in der zweiten Stellung angeordnet, also so weit in den ersten länglichen Hohlprofilkörper 2 eingeschoben, dass das Blockierelement 14, also das verdickte Ende des zweiten länglichen Hohlprofilkörpers 3, gegen das proximale Ende des ersten länglichen Hohlprofilkörpers 2 drückt. Auch die zweite Erhebung 10 dient als Blockierelement, das in dieser zweiten Stellung gegen das distale Schlitzende drückt. Der erste längliche Hohlprofilkörper 2 umfasst die Fingerringe 7, 8 und der zweite längliche Hohlprofilkörper 3 umfasst den Daumenring 6. Durch diese Ringe kann der zweite längliche Hohlprofilkörper 3 leicht von der ersten Stellung in die zweite Stellung geschoben und wieder in die erste Stellung gebracht werden.

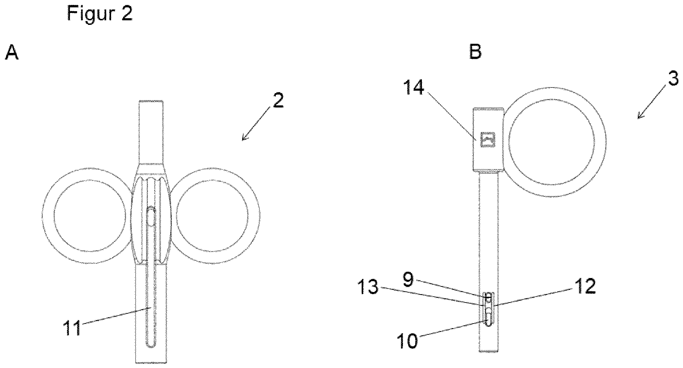

Fig. 2A zeigt den ersten länglichen Hohlprofilkörper 2 mit dem Schlitz 11, in dessen proximales Ende der zweite längliche Hohlprofilkörper 3 eingeschoben werden kann.

Fig. 2B zeigt den zweiten länglichen Hohlprofilkörper 3 mit dem Blockierelement 14 am proximalen Ende und der ersten Erhebung 9 und der zweiten Erhebung 10 an seiner Außenseite. Im Bereich dieser Erhebungen weist der zweite längliche Hohlprofilkörper 3 die Aussparungen 12, 13 auf, die für eine elastische Bewegungsmöglichkeit der Erhebungen 9, 10 sorgen.

### Bezugszeichenliste

- 1): Einführinstrument
- 2): erster länglicher Hohlprofilkörper
- 3): zweiter länglicher Hohlprofilkörper
- 4): Rohr
- 5): Olive
- 6): Daumenring
- 7): Fingerring
- 8): Fingerring
- 9): Erste Erhebung
- 10): Zweite Erhebung
- 11): Schlitz
- 12): Aussparung
- 13): Aussparung
- 14): Blockierelement

## Patentansprüche

1. Einführinstrument für Katheter umfassend
einen ersten länglichen Hohlprofilkörper mit einem distalen und einem proximalen Ende,
einen zweiten länglichen Hohlprofilkörper, der teilweise in den ersten länglichen Hohlprofilkörper eingeführt ist, der verschiebbar in dem ersten länglichen Hohlprofilkörper gelagert ist und der sich über das proximale Ende des ersten länglichen Hohlprofilkörpers hinaus erstreckt, und
ein sich an das distale Ende des ersten länglichen Hohlprofilkörpers anschließendes Rohr,
wobei der zweite längliche Hohlprofilkörper von einer ersten Stellung in eine zweite Stellung verschiebbar ist, in welcher der zweite längliche Hohlprofilkörper weiter in den ersten länglichen Hohlprofilkörper eingeführt ist als in der ersten Stellung,
wobei in der ersten Stellung ein weiteres Einführen des zweiten länglichen Hohlprofilkörpers in den ersten länglichen Hohlprofilkörpers gehemmt ist.

2. Einführinstrument nach Anspruch 1, wobei in der ersten Stellung ein weiteres Herausziehen des zweiten länglichen Hohlprofilkörpers aus dem ersten länglichen Hohlprofilkörper gehemmt ist.

3. Einführinstrument nach Anspruch 1 oder 2, wobei in der zweiten Stellung ein weiteres Einführen des zweiten länglichen Hohlprofilkörpers in den ersten länglichen Hohlprofilkörpers blockiert ist.

4. Einführinstrument nach einem der Ansprüche 1 bis 3, wobei die Hemmung in der ersten Stellung durch eine Hemmvorrichtung erreicht wird, die eine Wechselwirkung, bevorzugt eine Reibwirkung, zwischen dem ersten und dem zweiten länglichen Hohlprofilkörper erzeugt, vorzugsweise wobei die Hemmvorrichtung innerhalb des ersten länglichen Hohlprofilkörpers angeordnet ist.

5. Einführinstrument nach Anspruch 4, wobei die Hemmvorrichtung eine erste Erhebung auf einem der Hohlprofilkörper umfasst, die in der ersten Stellung gegen den anderen Hohlprofilkörper drückt, vorzugsweise wobei der Hohlprofilkörper, gegen den die erste Erhebung des anderen länglichen Hohlprofilkörpers drückt, eine Nut oder einen Schlitz zur Aufnahme der ersten Erhebung nach dem Überwinden eines Widerstands aufweist.

6. Einführinstrument nach Anspruch 5, wobei die erste Erhebung an dem zweiten länglichen Hohlprofilkörper angeordnet ist und/oder wobei die Nut oder der Schlitz an dem ersten länglichen Hohlprofilkörper angeordnet ist.

7. Einführinstrument nach einem der Ansprüche 1 bis 6, umfassend eine zweite Erhebung auf einem der Hohlprofilkörper, die in der ersten Stellung ein weiteres Herausziehen des zweiten länglichen Hohlprofilkörpers aus dem ersten länglichen Hohlprofilkörper hemmt, vorzugsweise wobei die zweite Erhebung distal zur ersten Erhebung angeordnet ist, vorzugsweise wobei die zweite Erhebung auf dem länglichen Hohlprofilkörper angeordnet ist, der die erste Erhebung aufweist.

8. Einführinstrument nach einem der Ansprüche 1 bis 7, wobei die erste und/oder die zweite Erhebung elastisch gelagert sind, bevorzugt wobei der längliche Hohlprofilkörper, der die jeweilige Erhebung aufweist, im Bereich dieser Erhebung eine oder mehrere Aussparungen aufweist.

9. Einführinstrument nach einem der Ansprüche 1 bis 8, wobei der zweite längliche Hohlprofilkörper ein Blockierelement aufweist, das in der zweiten Stellung ein weiteres Einführen in den ersten länglichen Hohlprofilkörper blockiert, vorzugsweise wobei das Blockierelement außerhalb des ersten länglichen Hohlprofilkörpers angeordnet ist.

10. Einführinstrument nach Anspruch 9, wobei das Blockierelement einen anderen, vorzugsweise einen größeren, Querschnitt aufweist, als der Teil des zweiten länglichen Hohlprofilkörpers, der vorgesehen ist, in den ersten länglichen Hohlprofilkörper eingeführt zu werden.

11. Einführinstrument nach einem der Ansprüche 1 bis 10, wobei der zweite längliche Hohlprofilkörper einen Daumenring zum leichteren Einführen des zweiten länglichen Hohlprofilkörpers in den ersten länglichen Hohlprofilkörper aufweist.

12. Einführinstrument nach einem der Ansprüche 1 bis 11, wobei der erste längliche Hohlprofilkörper mindestens einen Fingerring aufweist.

13. Einführinstrument nach einem der Ansprüche 1 bis 12, wobei der zweite längliche Hohlprofilkörper ausgestaltet ist, einen Katheter in sich aufzunehmen.

14. Einführinstrument nach Anspruch 13, wobei der zweite längliche Hohlprofilkörper ausgestaltet ist, einen aufgenommenen Katheter derart zu fixieren, dass dieser beim Verschieben des zweiten länglichen Hohlprofilkörpers relativ zum ersten länglichen Hohlprofilkörpers mitgeführt wird.

15. Einführinstrument nach einem der Ansprüche 1 bis 14, wobei das sich an das distale Ende des ersten länglichen Hohlprofilkörpers anschließende Rohr gekrümmt ist und/oder
eine Verjüngung zum distalen Ende hin aufweist und/oder
eine distale Olive aufweist, die vorzugsweise eine Markierung aufweist.
